# EUROPEAN PATENT SPECIFICATION

(11) **EP 0 662 108 B1**
(45) Date of publication and mention of the grant of the patent: **26.02.1997**
(21) Application number: 93920813.8
(22) Date of filing: 22.09.1993
(51) Int. Cl.: C09C 1/30, A61K 7/16

(54) **PROCESS FOR IMPROVING THE COMPATIBILITY OF PRECIPITATED SILICAS WITH FLAVOURS**
VERFAHREN ZUR VERBESSERUNG DER VERTRAEGLICHKEIT VON FAELLUNGSKIESELSAEUREN MIT AROMEN
PROCEDE POUR AMELIORER LA COMPATIBILITE DE SILICES PRECIPITEES AVEC DES AROMES

(30) Priority: 24.09.1992 GB 9220151
(43) Date of publication of application: 12.07.1995
(73) Proprietor: UNILEVER PLC, London EC4P 4BQ (GB); UNILEVER N.V., 3013 AL Rotterdam (NL)
(72) Inventor: BIJLANI, Nand, Sanmukhdas D-5 Mira Mansion, Bombay 400022 (IN)
(74) Representative: van Gent, Jan Paulus
(86) International application number: EP9302591
(87) International publication number: WO9406868

(56) References cited:
- US-A- 4 421 527
- CHEMICAL ABSTRACTS, vol. 100, no. 10, 5 March 1984, Columbus, Ohio, US; abstract no. 73801e, page 340 ;

## Description

The present invention relates to an improvement in the flavour compatibility of precipitated silicas, used in dental care products such as dentifrices.

In dental care products such as dentifrices various types of silicas are frequently used, for several purposes. They are mainly synthetic silicas, of which the three main types are pyrogenic silicas, precipitated silicas, and silica gels (aerogels, xerogels and hydrogels). These silicas are used as abrasive agents or as thickening agents in dentifrices. For imparting abrasive and thickening properties to dentifrices, particularly to gel-type dentifrices, precipitated silicas are the most frequently used. The present invention is particularly concerned with such precipitated silicas.

Precipitated silicas are commonly prepared by precipitating them from dilute sodium silicate solutions by the addition of mineral acid and sometimes salt, followed by washing with water and drying. They mainly differ from the pyrogenic silicas and silica gels in that they have a much smaller surface area (e.g. 150-450 m²/g) and a much larger pore volume (e.g. 0.4-1.0 ml/g) than the pyrogenic silicas or the silica gels (e.g. 600-800 m²/g and 0.2-0.4 ml/g). Also the average pore size is different (more than 2 nm compared with 1-1.2 nm)

Dental care products such as dentifrices almost invariably also contains flavours added thereto, but we have found that the impact of such flavours is much less pronounced in dentifrices that contain precipitated silicas than in dentifrices that contain one of the other types of synthetic silicas, e.g. abrasive silicas of the silica gel type.

It is therefore an object of the present invention to improve the flavour impact of flavours, present in a dentifrice that also contains a precipitated silica.

We have now surprisingly found, that this object can be achieved to a significant extent by improving the compatibility of such precipitated silicas with flavours. This improved compatibility is achieved by treating the precipitated silica with an alkaline material.

Consequently, in this broadest aspect the present invention relates to a process for improving the compatibility of precipitated silicas with flavours, wherein said silicas are treated with an alkaline material. A further object of the invention is to improve the foaming power and creaminess of the foam of a dentifrice that comprises a detergent-active material by inclusion therein of an alkali-treated precipitated silica, which produces a synergistic interaction with the detergent-active material.

In this respect it is observed, that in US Patent 4,421,527 a process is described for improving the compatibility of abrasive precipitated silicas with fluorides, whereby these silicas are treated with an alkaline earth metal compound. According to this reference the thus treated abrasive precipitated silicas contain silanol groups on their surface which are more readily available and have a higher surface acidity than similar materials made from sodium silicate/sodium sulfate solutions to which an alkaline earth metal salt has been added. The improved thickening precipitated silicas of the present invention however have a reduced surface acidity, silanol groups on their surface being less readily available.

It is also known from GB 2,022,410 and 2,080,784 to treat abrasive silicas of the hydrogel-type with an alkaline material to improve its abrasiveness, but as said above silicas of the gel-type do not suffer from serious flavour incompatibility problems.

In Chemical Abstracts, Vol. 100, No. 10, 1984, 73801e, Indian Patent 151,862 is referred to which describes a process for removing iron from silicas by the treatment of precipitated 3 silicas with alkali at pH8; prior to drying said silicas. There is no reference to so treating silicas after drying to improve their flavour-compatibility.

The treatment of the precipitated silicas with an alkaline material according to the present invention should take place after the precipitated silica has been dried. The dried precipitated silica can for instance be slurried with water and a suitable amount of an alkaline material can then be added to the slurry, whereupon the treated silica can be filtered off and dried. Although any conventional drying method may be used, spray-drying or flash drying is the preferred method to achieve a product which matches closely the original precipitated silica in terms of particle size and slurry viscosity.

The dried precipitated silica may also be added to a dentifrice formulation during the manufacture thereof, said formulation containing a suitable alkaline material, prior to the incorporation of a flavour to such dentifrice formulation. This can be done by in-situ treatment of the silica with the alkaline material in the presence of humectants during the dentifrice processing. Preferred, however, is the separate treatment of the dried silica with the alkaline material.

Suitable alkaline materials are sodium hydroxide, potassium hydroxide, sodium or potassium carbonate, sodium or potassium bicarbonate, sodium fluoride, tetrasodiumpyrophosphate, sodium trimetaphosphate, ammonia, organic amines and so on.

In general, such an amount of alkaline materials is used that a 10% aqueous slurry of the silica has a pH of between 7.0 and 10.0, preferably 7.0 and 8.5, preferably between 7.5 and 8.5 and particularly preferably between 8.0 and 8.5.

The alkali-treated precipitated silica of the invention is particularly useful in flavour-containing dental care products such as dentifrices. They are usually included therein in an amount of from 1-25%, commonly in an amount of 5-20% by weight. They are suitable for inclusion in both gel-type dentifrices as in non-gel type dentifrices. Both types of dentifrices may contain the usual additional ingredients in conventional amounts. Thus, as dentifrice it will usually comprise an abrasive cleaning agent in an amount of from 3-75% by weight. Suitable abrasive cleaning agents are milled or unmilled particulate aluminas; silica xerogels, hydrogels and aerogels; precipitated silica; calciumpyrophosphate; insoluble sodium metaphosphate; calcium carbonate; dicalcium orthophosphate; particulate hydroxyapatite and so on.

Furthermore, the dentifrice may contain a liquid phase comprising water and a humectant in an amount of 10-99% by weight. Typical humectants are glycerol, sorbitol, polyethyleneglycol, polypropylene glycol, propylene glycol, hydrogenated partially hydrolyzed polysaccharides and so on.

Binders or thickening agents such as sodium carboxymethylcellulose, hydroxyethylcellulose, hydroxypropylcellulose, xanthan gums, Irish moss, gum tragacanth, finely-divided hectorites may also be included in the dentifrice in an amount of 0.5 -10% by weight. Another conventional ingredient in a dentifrice is an organic surfactant such as a soap, an anionic, nonionic, cationic, ampholytic and/or a zwitterionic synthetic detergent surfactant in an amount of 0.2-5% by weight.

Various other optional ingredients may be included in the compositions of the invention, e.g. sweetening agents such as sodium saccharinate, whitening agents such as titanium dioxide or zinc oxide, preservatives, vitamins such as vitamin C and E, anti-plaque agents such as zinc salts like zinc citrate, copper salts, stannous salts like stannouspyrophosphate, sanguinarine, allantoin, p-aminobenzoic acid derivates, hexetidine, chlorhexidine, 3-(4-propylheptyl)- 4-(2-hydroxyethyl)- morpholine, antibacterial agents such as Triclosan (2',4,4'-trichloro-2-hydroxy-diphenyl ether), anti-calculus agents such as di- and/or tetra-alkalimetalpyrophosphates, pH adjusting agents, colouring agents, anti-caries agents such as casein, casein digests, sodium trimetaphosphate, sodium fluoride and monosodiumfluorophosphate, anti-staining compounds such as silicone polymers, anti-inflammatory agents such as substituted salicylanilides, plant extracts, desensitizing agents for sensitive teeth such as potassium chloride, potassium nitrate and potassium citrate, polymers such as polyvinylmethylether-maleic anhydride copolymers and so on.

Flavours to be used in the present invention can be any of the commonly used, well-known flavours for dentifrices such as peppermint oil, spearmint oil, menthol, carvone and so on. Flavours are usually used in an amount of 0.01 - 2%.

The present invention will now be further illustrated by the following Examples.

### Example 1

10 g of thickening precipitated silica (MFIL grade ex Madhu Silica) was taken in 90 ml of water. The slurry was stirred uniformly and initial pH was measured (pH = 6.3). Sodium hydroxide solution (0.2N, 8.5 ml) was added dropwise with continuous stirring of the slurry till the pH was adjusted to 8 - 8.5. The slurry was then filtered and dried till the moisture content of the dried silica was less than 5%. The dried lumps of silica were then ground to the desired particle size.

The compatibility of the thus treated thickening precipitated silica with flavours was tested in a model system with the flavour, used in a commercial dentifrice Close Up Blue, using Headspace GLC analysis.

Equilibrium head space gas chromatography was used to determine the vapor phase composition of flavour in equilibrium with the silica. The flavour-adsorbed silica in equilibrium with flavour vapours was taken into a head space vial of 50 ml capacity. The sealed vial was equilibrated at 50°C for 20 minutes. An aliquot of the head space was injected into the GC column. The chromatogram was processed by a computing integrator. The area given by the pure flavour was taken as standard and the results were normalised.

The following results were obtained:

| Sample | Weight of sample (in g) | % area (based on pure flavour) |
|---|---|---|
| neat flavour | 0.11 | 100.0 |
| | | |
| untreated thickening silica + flavour | 1.0+0.11 | 61.5 |
| | | |
| treated thickening silica + flavour | pH 7.5 1.0+0.11 | 94.4 |
| | | |
| treated thickening silica + flavour | pH 8.0 1.0+011 | 99.3 |

These results show, that with the alkali-treated thickening precipitated silica the flavour impact is almost the same as with the pure flavour, and significantly better than with the untreated thickening silica.

Temperature programmed desorption studies showed, that with the untreated thickening silica higher levels of energy for desorption were required, as the flavour is released in the temperature range of 220-400°C. With the alkali-treated thickening silica no peaks were observed in this temperature range.

### Example 2

10g of abrasive precipitated silica (AC-37 ex Crosfield U.K.) was taken in 90 ml water. The slurry was stirred uniformly and the initial pH was measured (pH 5.9). Sodium hydroxide solution (0.2N, 13 ml) was added dropwise with continuous stirring of the slurry till the pH was adjusted to 8-8.5. The slurry was filtered and dried as in Example 1. The compatibility of this treated abrasive precipitated silica with flavour was carried out as outlined in Example 1. The following results were obtained:

| Sample | Wt. of sample | % area (based on pure flavour) |
|---|---|---|
| Neat flavour | 0.11 | 100.0 |
| | | |
| Untreated abrasive precipitated silica + flavour | 1 + 0.11 | 80.0 |
| | | |
| Treated abrasive precipitated silica + flavour | 1 + 0.11 | 98.0 |

These results show that with the alkali treated abrasive precipitated silica, the flavour impact is almost the same as with pure flavour and significantly better than with untreated abrasive precipitated slilica.

### Example 3

Repeating the experiment of Example 1, but using the flavour of the commercial dentifrice Close Up red, gave the following results:

| Sample | Weight of sample (in g) | % area (based on pure flavour) |
|---|---|---|
| neat flavour | 0.12 | 100.0 |
| | | |
| abrasive silica (gel type) + flavour | 1 + 0.12 | 100.0 |
| | | |
| untreated thickening silica + flavour | 1 + 0.12 | 9.0 |

These results show, that with other types of silicas (e.g. gel type), the loss of flavour impact is only minimal, whereas the loss of flavour impact with an untreated thickening precipitated silica is quite significant.

### Example 4

### Comparison of a toothpaste formulation containing the modified thickening silica as prepared in Example 1, and a control formulation with an unmodified silica

| Ingredient | Wt. % | |
|---|---|---|
| | Control | Expt |
| Sorbitol (70 %) | 70.0 | 70.0 |
| Abrasive Silica | 10.00 | 10.0 |
| | | |
| Precipitated thickening silica (MFIL grade) unmodified | 8.00 | -- |
| | | |
| Modified MFIL according to Example 1 | -- | 8.0 |
| | | |
| Sodium carboxymethylcellulose | 0.6 | 0.6 |
| | | |
| Sodium Saccharin | 0.2 | 0.2 |
| | | |
| Flavour | 1.1 | 1.0 |
| | | |
| Polyethylene glycol (M.W. 1500) | 1.0 | 1.0 |
| | | |
| Sodium laurylsulphate | 2.5 | 2.5 |
| | | |
| Sodium dodecylbenzenesulphonate | 0.2 | 0.2 |
| | | |
| Colour, etc. | q.s. | q.s. |
| | | |
| Water to | 100.0 | 100.0 |

These formulations were assessed in a Qualitative Description Analysis (QDA) by an expert panel.

Qualitative descriptive analysis or QDA is a subjective assessment of specific product attributes of toothpastes. This is carried out by a panel consisting of 15-16 people trained to evaluate toothpaste performance. The product attributes assessed in this method are foam amount, foam creaminess, flavour impact, pungency, mouth freshness, cooling after brushing, paste spreadability, grittiness, etc. The protocol for brusing involves taking half a brush length of the sample on a pre-wetted brush and brushing for 60 seconds. Panelists are asked to assess each of the attributes and rate them on a ten point scale. The mean rating for each attribute is calculated and plotted on a QDA chart, whereby comparative evaluation of overall performance of toothpaste samples can be done.

### Findings

The results are given in Table I. The performance of the experimental formulation was found to be comparable to the control formulation. The above findings show that the experimental formulation with a lower flavour level has comparable flavour impact to that of control.

It can also be seen from the data that foam creaminess is better in the experimental formulation as compared to control formulation.

The example shows that the toothpaste composition containing modified thickening silica has a synergistic effect with the flavour and detergent giving rise to better foam and flavour impacts.

| | **Mouth fresh** | **Freshness** | **Flavour** | **Pungency** | **Grittiness** | **Smooth on brush** |
|---|---|---|---|---|---|---|
| **Experimental Formulation** | 5.6 | 5 | 5 | 5 | 1.2 | 3.8 |
| **Control** | 5.5 | 5 | 5 | 5 | 1 | 4.7 |

| | **Ease to Squeeze** | **Creaminess** | **Foam** | **Cooling** | **Chalky Feeling** | **Spreadability** |
|---|---|---|---|---|---|---|
| **Experimental Formulation** | 4.6 | 6 | 6.2 | 5.5 | 1.3 | 5.4 |
| **Control** | 5.8 | 5.4 | 6 | 5.5 | 1.5 | 5.8 |

## Claims

1. A process for improving the compatibility of precipitated silicas with flavours, comprising treating dried precipitated silicas with an alkaline material.

2. The process of claim 1, wherein the alkaline material is sodium hydroxide.

3. The process of claim 1, wherein the dried precipitated silicas are treated with the alkaline material in an aqueous slurry, which is subsequently filtered off, after which the treated silicas are spray-dried.

4. A process for the preparation of a flavoured dentifrice composition comprising mixing the ingredients of the dentifrice composition, wherein the ingredients are admixed with a flavour and a dried precipitated silica which has been treated with an alkaline material according to anyone of claims 1-3.

5. A process according to claim 4, wherein a gel-type dentifrice composition is prepared containing a gel-type abrasive silica and an alkali-treated dried precipitated thickening silica.

## Patentansprüche

1. Verfahren zur Verbesserung der Verträglichkeit gefällter Siliciumdioxidsorten gegenüber Geschmackstoffen durch Behandeln von getrockneten, gefällten Siliciumdioxidsorten mit einem alkalischen Material.

2. Verfahren nach Anspruch 1, wobei das alkalische Material Natriumhydroxid ist.

3. Verfahren nach Anspruch 1, wobei die getrockneten, gefällten Siliciumdioxidsorten mit dem alkalischen Material in einer wäßrigen Aufschlämmung, die anschließend abgefiltert wird, behandelt werden, worauf die behandelten Siliciumdioxidsorten sprühgetrocknet werden.

4. Verfahren zur Herstellung einer Geschmackstoffe aufweisenden Zahnpastazusammensetzung durch Vermischen der Bestandteile einer Zahnpastazusammensetzung, wobei die Bestandteile mit einem Geschmackstoff und einem getrockneten, gefällten Siliciumdioxid, das mit einem alkalischen Material nach einem der Ansprüche 1 bis 3 behandelt worden ist, vermischt werden.

5. Verfahren nach Anspruch 4, wobei eine Zahnpastazusammensetzung vom Geltyp, die ein als Abriebmittel dienendes Siliciumdioxid vom Geltyp und ein alkalibehandeltes, getrocknetes, gefälltes, verdickendes Siliciumdioxid enthält, hergestellt wird.

## Revendications

1. Procédé pour améliorer la compatibilité des silices précipitées avec les arômes, comprenant le traitement des silices précipitées séchées avec une matière basique.

2. Procédé selon la revendication 1, dans lequel la matière basique est l'hydroxyde de sodium.

3. Procédé selon la revendication 1, dans lequel les silices précipitées séchées sont traitées avec la matière basique dans une suspension aqueuse, qui est ultérieurement éliminée par filtration, après quoi les silices traitées sont séchées par pulvérisation.

4. Procédé pour la préparation d'une composition dentifrice aromatisée consistant à mélanger les ingrédients de la composition de dentifrice, dans lequel on mélange les ingrédients avec un arôme et une silice précipitée séchée que l'on a traitée avec une matière basique selon l'une quelconque des revendications 1 à 3.

5. Procédé selon la revendication 4, dans lequel on prépare une composition de dentifrice contenant une silice abrasive du type gel et une silice épaississante précipitée séchée traitée avec une base.
